# EUROPEAN PATENT APPLICATION

(11) **EP 3 220 301 A1**
(43) Date of publication of application: **20.09.2017**
(21) Application number: 17161324.3
(22) Date of filing: 16.03.2017
(51) Int. Cl.: G06F 19/00

(54) **METHOD AND SYSTEM FOR COLLECTING AND ANALYSING DATA FROM HEALTH MONITORING DEVICES**

(30) Priority: 16.03.2016 US 201662308864 P
(71) Applicant: CRF Inc., Plymouth Meeting, PA 19462 (US)
(72) Inventor: STROBRIDGE, Richard, Alpine, CA 91901 (US)
(74) Representative: Berggren Oy, Helsinki & Oulu

(57) **Abstract**

In an event of disconnection of a local power network (2) from a grid (1), the change of the control mode of a generator set in the local power network from a kW mode (31) to a speed droop.

## Description

### Field of technology

The present invention relates generally to data informatics and analysis and more particularly to a system and method for the collection and analysis of data from health monitoring devices to produce alerts for improving a user's lifestyle.

### Prior art

With advancements in wireless technologies, home health monitoring has become more commonplace. These advances facilitate daily monitoring from a network of caregivers including family, friends, and clinicians. The health monitoring devices collect real-time data that may be stored and/or analyzed by the network or caregivers.

A large majority of those taking advantage of home health monitoring suffer from chronic diseases ranging from congestive heart failure and hypertension to chronic obstructive pulmonary disease (COPD) and diabetes. The real-time data collected by health monitoring devices provides invaluable insight and information to the caregivers about the quality of daily life experienced by the user. Based on this wealth of knowledge, clinicians and caregivers are equipped to recommend changes to increase the quality of daily life of the user.

The advances in wireless technologies have also resulted in a convergence of health monitoring devices, smartphones, information technology, and data informatics. While this convergence has the ability to revolutionize healthcare, a platform is needed that effectively connects the various technologies in a way that automates the process on an individualized basis. For example, by comparing real-time data collected by health monitoring devices with the historical data of the particular user and/or a network of user's, clinicians are better equipped to make relevant recommendations to aptly address the particular user's quality of life.

### Short description

The present invention overcomes these and other deficiencies of the prior art by providing a system and method for connecting a plurality of health monitoring devices and comparing the real-time data gathered from these devices with patient historical data to automatically provide remedial actions to be taken by a particular patient. Connection to the system of the present invention may be via a wired or wireless connection via the internet or cloud computing resources. The system collects data from the plurality of connected devices and sends it to a network of the system for analysis and comparison with the user's historical data and other user's data that is stored in a plurality of databases. The system then computes output data based on the user's current status in the form of a message alert recommending a remedial action for the individual user. The message may be sent to the user via a smartphone, tablet, desktop computer, and/or laptop computer.

The foregoing, and other features and advantages of the invention, will be apparent from the following, more particular description of the preferred embodiments of the invention, the accompanying drawings, and the claims.

### List of figures

For a more complete understanding of the present invention, and the advantages thereof, reference is now made to the ensuing descriptions taken in connection with the accompanying drawings briefly described as follows:
Fig. 1 illustrates an overall depiction of the system and method according to an embodiment of the invention;
Fig. 2 illustrates an exemplary block diagram of the system and method with connected health monitoring devices according to an embodiment of the invention;
Fig. 3 illustrates a flow chart of the process of sending collected data from a glucose device to the system according to an embodiment of the present invention;
Fig. 4 illustrates an example of the data collected and stored by a glucose device according to an embodiment of the invention;
Figs. 5A, 5B, and 5C illustrate flow charts of the process of collecting data from connected devices, analysing the data, and producing a message to be sent to the user based upon the user's current status according to an embodiment of the invention;
Fig. 6 illustrates an example of the data collected and stored by the system and method from connected devices according to an embodiment of the invention;
Fig. 7 illustrates an example of the data stored in the system status database for a patient using a connected glucose and CPAP device according to an embodiment of the invention;
Fig. 8 illustrates an example of the data stored in the system result database from which messages are extracted depending upon the current status of a user according to an embodiment of the invention; and
Fig. 9 illustrates a flow chart of the process of updating the system status and result database according to an embodiment of the invention.

### Description of the invention

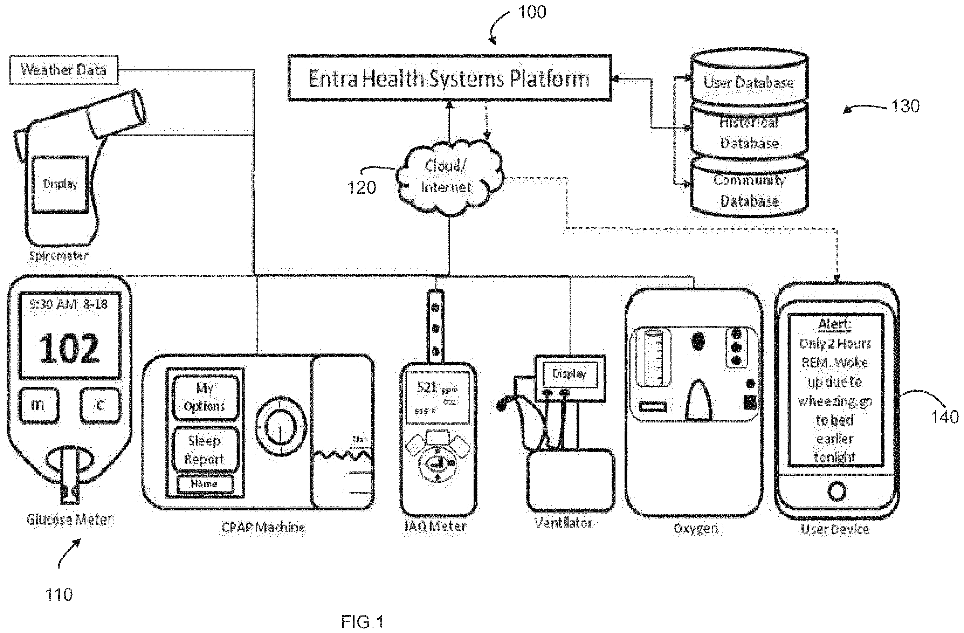

Preferred embodiments of the present invention and their advantages, as well as the operation of various embodiments of the invention are described in detail below with reference to the accompanying Figs. 1-9, wherein like reference numerals refer to like elements. Although the invention is described in the context of health monitoring devices, one of ordinary skill in the art readily appreciates that the system and methods described herein are applicable to any activity where the collection and analysis of data to generate real-time recommendations is warranted.

While the system and methodology described herein is extensible to health monitoring devices generally, for ease and the sake of clarity, the use case described herein with respect to the health monitoring device in the system and method of the present invention will be described in the context of a patient using a glucose meter and continuous positive airway pressure (CPAP) machine.

The system described herein is characterized by the ability to facilitate inclusion and/or connectivity with a plurality of connected devices. The connected devices may be connected to and in communication with the system via a wired connection or wireless connection. The wireless connection may be facilitated by known wireless protocols including, by way of non-limiting examples, infrared, Bluetooth, ZigBee, Wi-Fi, 3G/4G wireless protocols, radio frequency identification (RFID), and near field communication (NFC) protocols. In a preferred embodiment, the connected devices are health monitoring devices including, but not limited to, spirometer, glucose meter, CPAP machine, indoor air quality (IAQ) meter, ventilator, pulse oximeter, sphygmomanometer, thermometer, nebulizers, heart monitors, and the like. In further embodiments of the present invention the connected devices, in addition to the health monitoring devices include, by way of non-limiting examples, smartphones, tablets, desktop computers, laptop computers, and the like.

Referring to Fig. 1 is a broad depiction of the system 100 and method according to an embodiment of the invention. Connected devices 110 may include a plurality of health monitoring devices. Connection to the system of the present invention may be via a wired or wireless connection via the internet or cloud computing resources 120. The system 100 collects data from the plurality of connected devices 110. The collected data is sent to a network of the system 100 for analysis and comparison with the user's historical data and other user's data that is stored in a plurality of databases 130. The system 100 then computes output data based on the user's current status in the form of a message alert recommending a remedial action for the individual user. The message may be sent to the user via a smartphone, tablet, desktop computer, and/or laptop computer 140.

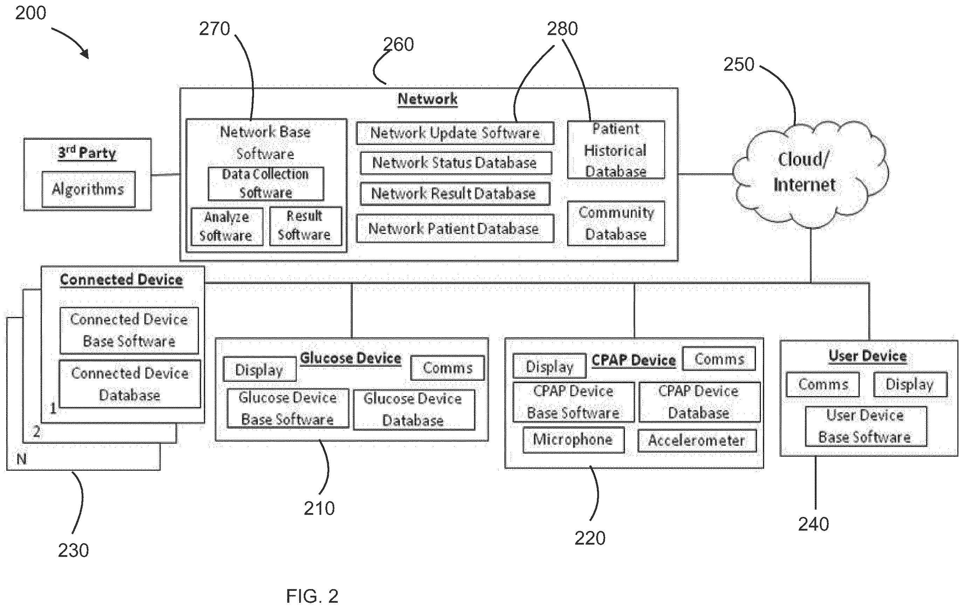

Fig. 2 illustrates an exemplary block diagram of the system 200 and method with connected health monitoring devices according to an embodiment of the invention. A glucose device 210, CPAP device 220, a plurality of connected devices 230, and a user device 240 are connected to the system 200 via the internet or cloud computing resources 250. Each of the connected devices includes its own internal suite of operational hardware modules and software programs. The internal hardware modules and software programs of each connected device collect and store data according to its programming. The glucose device 210, CPAP device 220, and plurality of connected devices 230 connect to the system and send the collected and stored data to the network of the system 260. The network of the system 260 may include a plurality of software programs 270 and a plurality of databases 280. For example, the plurality of software programs may include updating software; software to collect the data from the connected devices; software to analyse the collected data with historical data of the user and data from a community database; and software to compare the user's status with a results database to determine appropriate remedial actions for the user. The plurality of databases may include a patient/user database, a patient/user historical database, a community database, a status database, and a results database.

The network of the system 260 upon receiving the collected data stores the data in the requisite patient/user database. The analytics software then compares the collected data from the patient/user database to the status database. The status database includes predetermined ranges of measurables read from the various connected devices. The ranges of measurables are assigned a status, e.g., normal, caution, or high. A user's collected data can be compared to the ranges of measurables to determine a status for that user's particular read measurable from a particular connected health monitoring device. For example, a glucose meter may measure levels of glycated hemoglobin (HbA1c), mean blood, and glucose in a user. For purposes of this example, the glucose meter returns a measured reading for a user of 5 for HbA1c. The predetermined ranges for HbA1c in the status database for this user may be: 4 - 6 = Normal; 7 - 8 = Caution; and 9 - 14 = High. This collected data would be sent to the network of the present invention for comparison to the ranges of HbA1c in the status database by the analytics software. Therefore, the status for this particular user's HbA1c reading is Normal.

Once the analytics software extracts the status from the status database, the status is sent to the result software for comparison to the predetermined message associated with that particular status in the result database. The result software extracts the predetermined message associated with a particular status and sends the message alert to the user for appropriate remedial action if warranted based on the user's particular status.

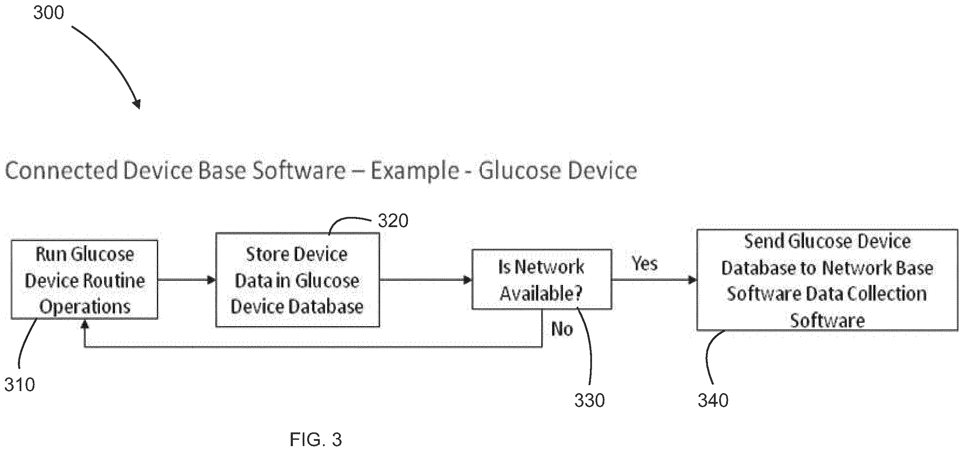

Fig. 3 illustrates a flow chart of the process of sending collected data from a glucose device to the system 300 according to an embodiment of the present invention. At 310 the internal suite of hardware modules and software programs for a glucose meter collect data from a user based upon the glucose meters programming. At 320 the collected data is stored in the internal database of the glucose meter. At 330, the glucose meter determines whether a connection to the network of the system 300 is available. If a connection exists, then at 340 the glucose meter sends the collected and stored data to the network of the system 300 for appropriate action by the system's software.

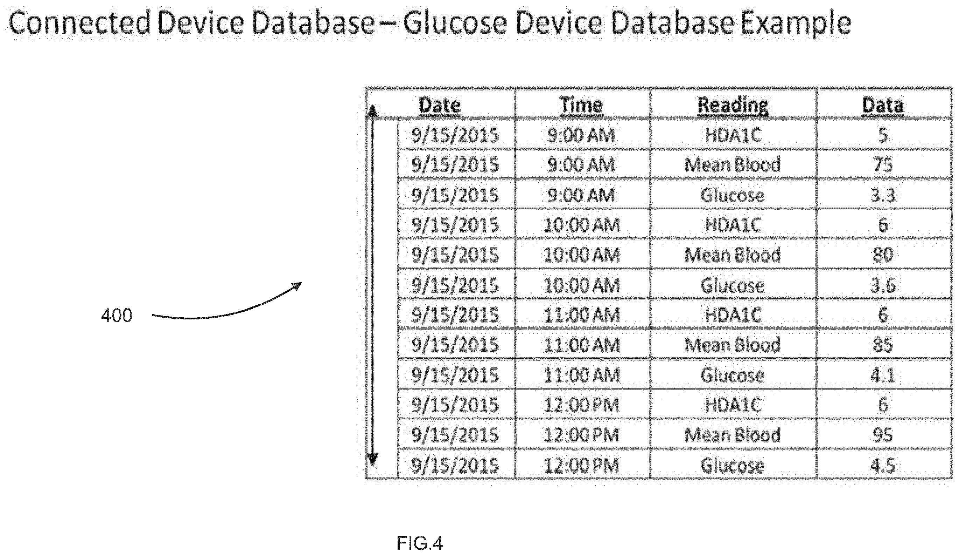

Fig. 4 illustrates an example of the data collected and stored 400 by a glucose device according to an embodiment of the invention. In this example, a glucose meter measures and collects data points at scheduled times during a day for HbA1 c, mean blood, and glucose. The data points are then stored in the internal database of the glucose meter for sending to the network of the system of the present invention for appropriate action by the system's software.

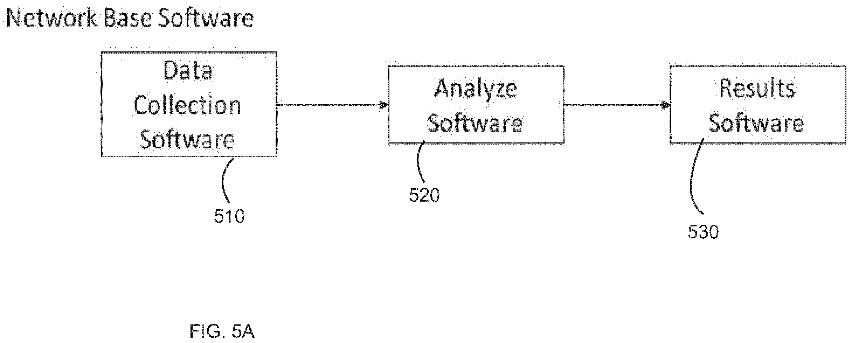

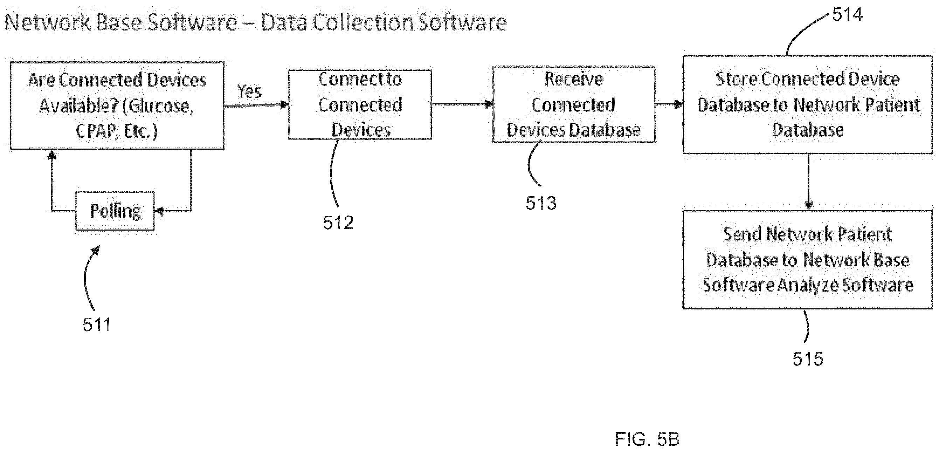

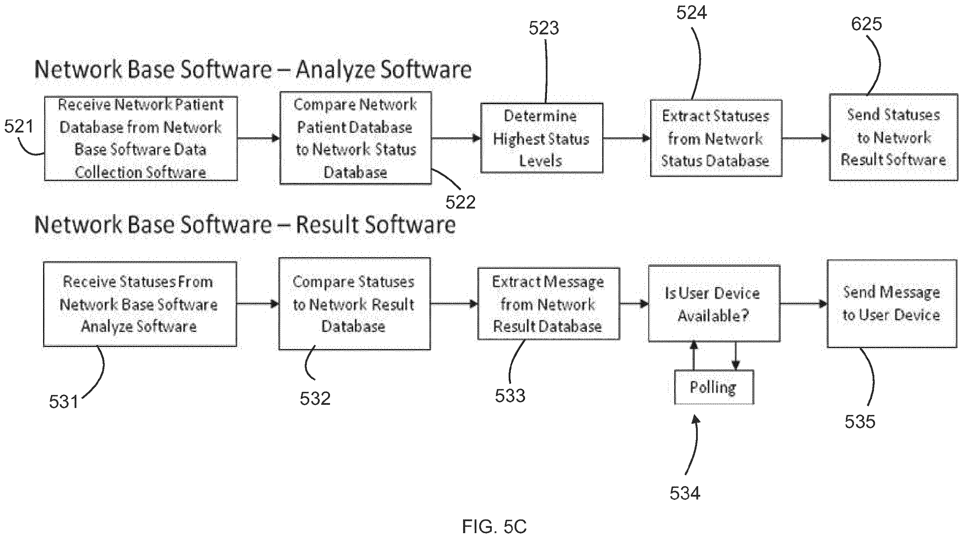

Figs. 5A, 5B, and 5C illustrate flow charts of the process of collecting data from connected devices, analysing the data, and producing a message to be sent to the user based upon the user's current status according to an embodiment of the invention. In Fig. 5A, the process 500 is an overall process flow once collected data is received from the connected devices by the system. At 510 the data collection software of the system receives the collected data from the plurality of connected devices and stores the collected data in the patient database. At 520 the data collection software sends the collected data to the analytics software of the system for comparison to the predetermined information in the status database. The analytics software extracts the appropriate status from the status database and sends it to the results software at 530. The results software compares the extracted status to the predetermined messages in the results database and extracts the associated message for sending to the user.

Fig. 5B provides a more detailed process flow once the collected data is received by the network of the system. At 511, the data collection software continuously polls for a connection to a connected device. If the data collection software finds an available connection to a particular device, then at 512, the data collection software connects to that particular device. At 513, the data collection software receives the collected and stored data from the connected device. At 514, the data collection software stores the collected data from the connected device in the system's patient database. At 515, the data collection software sends the collected data to the system's analytics software.

Fig. 5C provides a more detail process flow for the analytics and results software. At 521, the analytics software receives the collected data the data collection software. The collected data may be from one connected device or from a plurality of connected devices. At 522, the collected data is compared to the predetermined information in the status database. The analytics software at 523 determines the most critical status indicated based on the data from the connected devices. At 524, the most critical statuses are extracted from the status database. For example if the user had a measured reading from a glucose meter of 12, then from the example above in the description of Fig. 2, the status would be High and would be selected for extraction from the status database. At 525, the extracted statuses are sent to the result software for appropriate action.

The result software receives the statuses from the analytics software at 531. The statuses received by the result software are compared at 532 to the result database, which includes predetermined messages associated with a particular status of the user. At 533, the corresponding message associate with the extracted statuses is selected from the result database. At 534, the result software polls to determine if a user device is available to receive the selected message associate with a particular status of the user. If a user device is available, then at 535 the result software connects to the user device and sends the selected message as an alert to the user for appropriate remedial action.

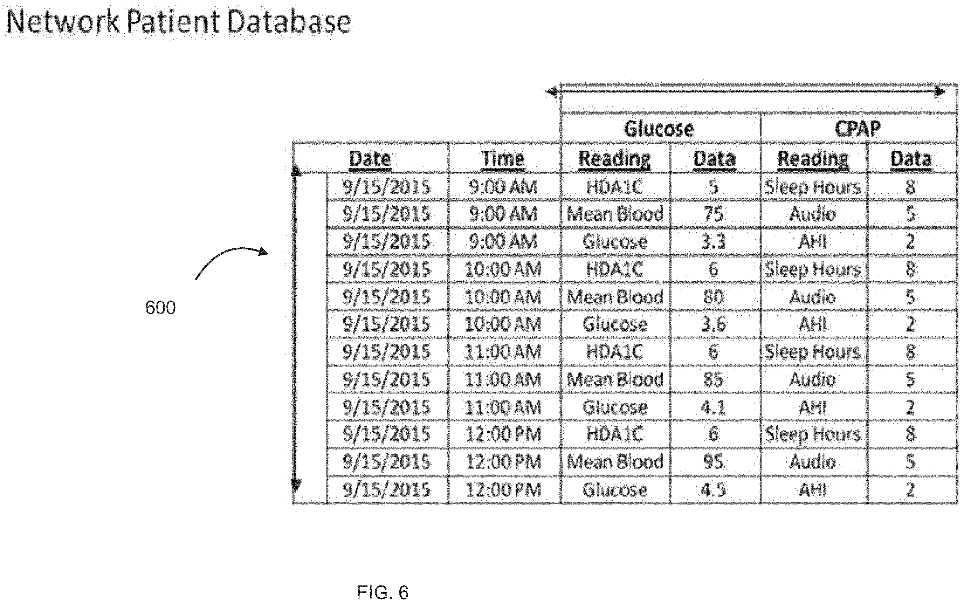

Fig. 6 illustrates an example of the data collected and stored 600 by the system and method from connected devices according to an embodiment of the invention. In this example, the data collected and stored 600 by the data collection software of the system is from a glucose meter and CPAP machine. The collected data 600 is stored in the patient database of the system and subsequently sent to the system's analytics software for appropriate action as described herein.

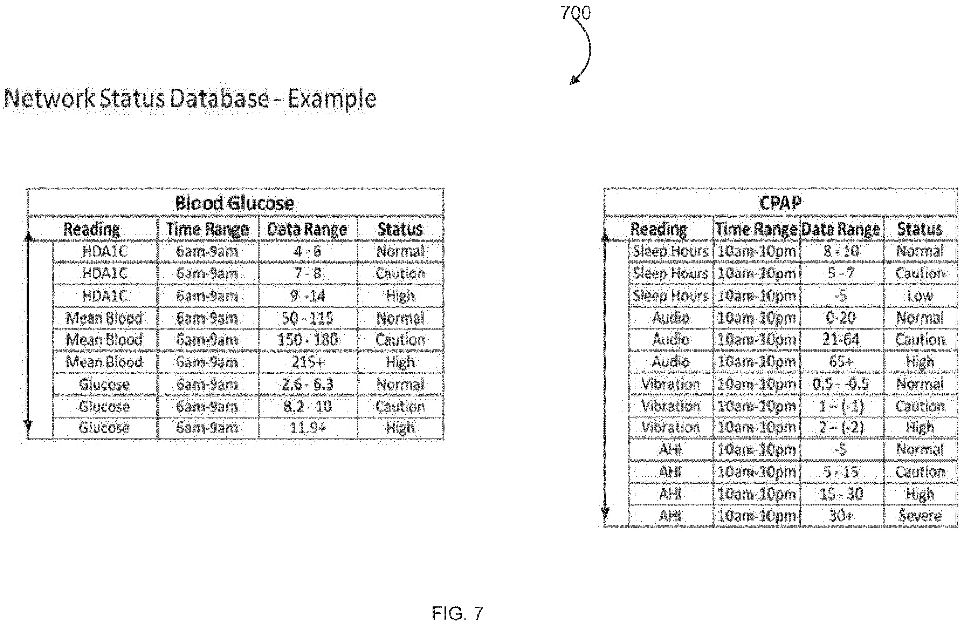

Fig. 7 illustrates an example of the data stored in the system status database 700 for a patient using a connected glucose and CPAP device according to an embodiment of the invention. The status database includes predetermined ranges of measurables from a plurality of connected device. In this exemplary status database, predetermined ranges of measurables from a glucose meter (HbA1c, mean blood, and glucose) and a CPAP machine (sleep hours, audio, vibration, and AHI) are stored. Each range of the measurables from the connected devices is assigned a status, i.e., Normal, Caution, High, Low, and/or Severe. The analytics software compares the collected data points from the connected devices to determine the highest priority or most critical status of a particular user, which is then sent to the results software for appropriate action.

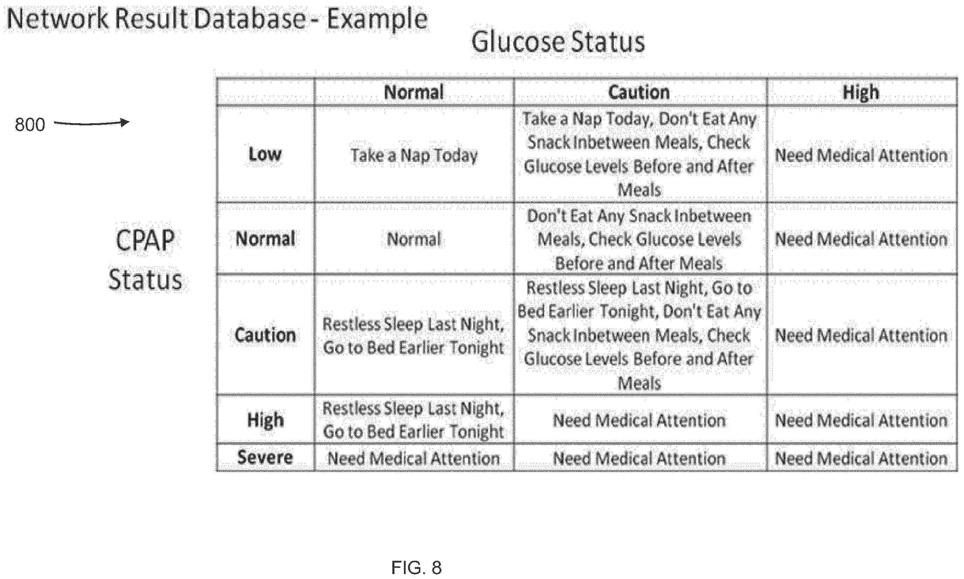

Fig. 8 illustrates an example of the data stored in the system result database 800 from which messages are extracted depending upon the current status of a user according to an embodiment of the invention. In this exemplary results database, predetermined messages associated with a particular status of a user's collected data is stored. For example, if the highest priority or most critical status of a particular user's glucose meter and CPAP machine readings is High and Severe, respectively, then the associated message indicated for retrieval by the results software is: Need Medical Attention. The results software would extract this message from the results database and send to a connected device of the user, which may be a health monitoring device, smartphone, laptop computer, desktop computer, and/or tablet computer and the like.

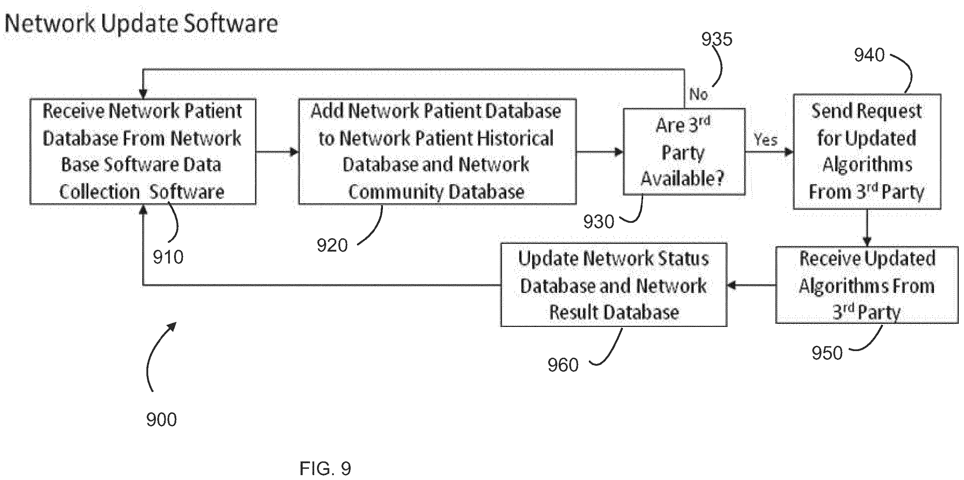

Fig. 9 illustrates a flow chart of the process of updating the system status and result database according to an embodiment of the invention. The system of the present invention includes software for constantly updating the plurality of databases in the system and updating the analytics and results software of the system. At 910, the update software receives the collected data in the patient database and adds, at 920, the data to the system's patient historical database and community database. In this embodiment, the update software, at 930, if third party algorithms are available to update the status and result databases. The third party algorithms contain the requisite predetermined ranges of measurables and associate status for the status database and the predetermined messages associated therewith for the results database. In this embodiment, at 940, if third party algorithms are available then a request is sent for updated algorithms. If no third party algorithms are available, then at 935, the process returns to receiving collected data from the patient database. At 950, the updated third party algorithms are received, and at 960 the status and results database are updated accordingly. It is to be understood that the update software runs in the background of the system and does not affect the process flow as described above with respect to Figs. 5A, 5B, and 5C.

Those of skill in the art will appreciate that the various illustrative logical blocks, modules, units, and algorithm steps described in connection with the embodiments disclosed herein can often be implemented as electronic hardware, computer software, or combinations of both. To clearly illustrate this interchangeability of hardware and software, various illustrative components, blocks, modules, and steps have been described above generally in terms of their functionality. Whether such functionality is implemented as hardware or software depends upon the particular constraints imposed on the overall system. Skilled persons can implement the described functionality in varying ways for each particular system, but such implementation decisions should not be interpreted as causing a departure from the scope of the invention. In addition, the grouping of functions within a unit, module, block, or step is for ease of description. Specific functions or steps can be moved from one unit, module, or block without departing from the invention.

The various illustrative logical blocks, units, steps and modules described in connection with the embodiments disclosed herein, and those provided in the accompanying documents, can be implemented or performed with a processor, such as a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein, and those provided in the accompanying documents. A general-purpose processor can be a microprocessor, but in the alternative, the processor can be any processor, controller, microcontroller, or state machine. A processor can also be implemented as a combination of computing devices, for example, a combination of a DSP and a microprocessor, a plurality of microprocessors, one or more microprocessors in conjunction with a DSP core, or any other such configuration.

The steps of a method or algorithm and the processes of a block or module described in connection with the embodiments disclosed herein, and those provided in the accompanying documents, can be embodied directly in hardware, in a software module executed by a processor, or in a combination of the two. A software module can reside in RAM memory, flash memory, ROM memory, EPROM memory, EEPROM memory, registers, hard disk, a removable disk, a CD-ROM, or any other form of storage medium. An exemplary storage medium can be coupled to the processor such that the processor can read information from, and write information to, the storage medium. In the alternative, the storage medium can be integral to the processor. The processor and the storage medium can reside in an ASIC. Additionally, device, blocks, or modules that are described as coupled may be coupled via intermediary device, blocks, or modules. Similarly, a first device may be described a transmitting data to (or receiving from) a second device when there are intermediary devices that couple the first and second device and also when the first device is unaware of the ultimate destination of the data.

The above description of the disclosed embodiments, and that provided in the accompanying documents, is provided to enable any person skilled in the art to make or use the invention. Various modifications to these embodiments will be readily apparent to those skilled in the art, and the generic principles described herein, and in the accompanying documents, can be applied to other embodiments without departing from the spirit or scope of the invention. Thus, it is to be understood that the description and drawings presented herein, and presented in the accompanying documents, represent particular aspects and embodiments of the invention and are therefore representative examples of the subject matter that is broadly contemplated by the present invention. It is further understood that the scope of the present invention fully encompasses other embodiments that are, or may become, obvious to those skilled in the art and that the scope of the present invention is accordingly not limited by the descriptions presented herein, or by the descriptions presented in the accompanying documents

## Claims

1. A method for collecting and analysing data from at least one health monitoring device, **characterised in that** the method comprises steps
to connect at least one health monitoring device with a network of a system;
to collect data from at least one connected health monitoring device and to store the collected data,
to compare the collected data with a user's historical data and other user's data in order to determine status or statuses for the user,
to compare the status to predetermined messages in order to extract a predetermined message/s associated with the status or statuses.

2. A method according to Claim 1, **characterised in that** the method further comprises a step to send the predetermined message/s to the user.

3. A method according to Claim 1 or 2, **characterised in that** the comparison step of the collected data is arranged to compare the collected data with predetermined ranges, each range being associated with a range specific status, and to determine the most critical status or statuses.

4. A method according to any of Claim 1 - 3, **characterised in that** the method further comprises a step to poll connections before the connection step.

5. A method according to any of Claim 2 - 4, **characterised in that** the method further comprises a step to poll an available user device for the step to send the predetermined message to the user.

6. A method according to any of Claim 1 - 5, **characterised in that** the method further comprises a step to update a system providing the steps said in any of claim 1-6.

7. A method according to any of claim 1 - 7, **characterised in that**
the method is implemented with at least one processor.

8. A storage medium of software arranged to be with at least one processor, **characterised in that** the storage medium of the software is arranged to provide steps for collecting and analysing data from at least one health monitoring device, which steps are arranged to
to connect at least one health monitoring device with a network of a system;
to collect data from at least one connected health monitoring device and to store the collected data,
to compare the collected data with a user's historical data and other user's data in order to determine status or statuses for the user,
to compare the status to predetermined messages in order to extract a predetermined message/s associated with the status or statuses.

9. A method according to Claim 8, **characterised in that** the method further comprises a step to send the predetermined message/s to the user.

10. A method according to Claim 8 or 9, **characterised in that** the comparison step of the collected data is arranged to compare the collected data with predetermined ranges, each range being associated with a range specific status.

11. A method according to Claim 10, **characterised in that** the comparison step of the collected data is arranged to determine the most critical status or statuses.

12. A method according to any of Claim 9 - 11, **characterised in that** the method further comprises a step to poll connections before the connection step.

13. A method according to any of Claim 9 - 12, **characterised in that** the method further comprises a step to poll an available user device for the step to send the predetermined message to the user.

14. A method according to any of Claim 9 - 13, **characterised in that** the method further comprises a step to update a system providing the steps said in any of claim 1-6.

15. A system to collect and analyse data from at least one health monitoring device, **characterised in that** the system comprises modules and databases for storing data, the modules being arranged
to connect at least one health monitoring device with the system;
to collect data from at least one connected health monitoring device and to store the collected data in the databases,
to compare the collected data with a user's historical data and other user's data in the databases in order to determine status or statuses for the user,
to compare the status to predetermined messages in the databases in order to extract a predetermined message/s associated with the status or statuses.

16. A system according to Claim 15, **characterised in that** the modules are further arranged to send the predetermined message to the user.

17. A system according to Claim 15 or 16, **characterised in that** said comparison of the collected data is arranged to compare the collected data with predetermined ranges, each range being associated with a range specific status.

18. A system according to Claim 17, **characterised in that** said comparison of the collected data is arranged to determine the most critical status or statuses.

19. A system according to any of Claim 15 - 18, **characterised in that** the modules are further arranged to poll connections before the connection step.

20. A system according to any of Claim 16 - 19, **characterised in that** the modules are further arranged to poll an available user device for the step to send the predetermined message to the user.

21. A system according to any of Claim 15 - 20, **characterised in that** the modules are further arranged to update a system providing said actions of the modules.

22. A system according to any of Claim 15 - 21, **characterised in that** the databases comprises a status database for the statuses, a result databases for the predetermined messages, and patient databases for the user's historical data and the other user's data.

23. A system according to Claim 22, **characterised in that** the modules comprises a data collection module to collect said data from at least one health monitoring device, an analyse module to compare the collected data with a user's historical data and other user's data in the databases in order to determine status or statuses for the user and a result module to compare the status to predetermined messages in the databases in order to extract a predetermined message/s associated with the status or statuses.

24. A patient device being a health monitoring device, smartphone, tablet, desktop computer, laptop computer, and the like, **characterised in that** the patient device is arranged to be a part of a system according to any of claim 15 - 23.
